(19) Europäisches Patentamt
European Patent Office
Office européen des brevets

(11) **EP 4 174 735 A1**

(12) **EUROPEAN PATENT APPLICATION**
published in accordance with Art. 153(4) EPC

(43) Date of publication:
**03.05.2023 Bulletin 2023/18**

(21) Application number: **20942832.5**

(22) Date of filing: **30.06.2020**

(51) International Patent Classification (IPC):
***G06N 20/00*** (2019.01)

(52) Cooperative Patent Classification (CPC):
**G06N 20/00**

(86) International application number:
**PCT/JP2020/025646**

(87) International publication number:
**WO 2022/003816 (06.01.2022 Gazette 2022/01)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **FUJITSU LIMITED
Kawasaki-shi, Kanagawa 211-8588 (JP)**

(72) Inventors:
• **TAKAGI, Takuya
Kawasaki-shi, Kanagawa 211-8588 (JP)**
• **KOBAYASHI, Ken
Kawasaki-shi, Kanagawa 211-8588 (JP)**
• **KANAMORI, Kentaro
Sapporo-shi, Hokkaido 060-0814 (JP)**

(74) Representative: **Hoffmann Eitle
Patent- und Rechtsanwälte PartmbB
Arabellastraße 30
81925 München (DE)**

(54) **INFORMATION PROCESSING PROGRAM, PROPOSITION METHOD, AND INFORMATION PROCESSING DEVICE**

(57)     An information processing apparatus (10) obtains a plurality of attribute datasets (1) including a subject attribute dataset, which are each a combination of a plurality of item values, and information indicating an area range that is taken as a target in a dimensional space (3). The information processing apparatus (10) then performs a determination process of determining a change-target item value to be changed from among the plurality of item values of the subject attribute dataset and a changing direction for the change-target item value on the basis of the distribution of at least some of the plurality of attribute datasets (1) in the dimensional space (3). The information processing apparatus (10) outputs information indicating a result of changes made by alternately iterating a changing process of changing the change-target item value and the determination process that follows the changing process until the subject attribute dataset is within the area range.

FIG. 1

EP 4 174 735 A1

## Description

Technical Field

The embodiments discussed herein relate to an information processing program, a recommendation method, and an information processing apparatus.

Background Art

Computer-based machine learning may be performed to create a model of receiving, as input values, a plurality of item values about a prediction target such as an event, a thing, or a person and predicting a class to which the prediction target belongs. In this case, there may arise a desire to know which item values of the prediction target and how much the item values need to be changed in order to change the class to which the prediction target belongs. For example, a person who is currently expected to be unhealthy from a result of medical checkup would like to know which check items and how much the values of them need to be improved so that he/she will be expected to be healthy. To satisfy this need, there is an idea to make a recommendation as to appropriate item values to be changed using a computer.
As a technique for making a recommendation for changing a prediction result, for example, there has been proposed an action quantity generation apparatus that efficiently finds how and how much an explanatory variable of prediction target data needs to be changed in order to change the prediction result to a desired value.

Citation List

Patent Literature

[PTL1] Japanese Laid-open Patent Publication No. 2003-288580

Summary of Invention

Technical Problem

However, a recommendation as to an item value to be changed and a change amount for the item value may be unrealistic and not feasible. Assume, for example, a scatter plot in which data points corresponding to a plurality of elements included in training data used for creating a model are plotted at positions corresponding to a plurality of item values of the elements. When an item value of a prediction target element is updated in accordance with a recommendation, the data point corresponding to the prediction target element may move through an area where other data points do not exist. The area where the data points corresponding to other elements do not exist is an area where a combination of item values belonging to this area is impossible or very unlikely. For this reason, a recommendation of changing an item value such that its corresponding data point moves through an area where the data points corresponding to other elements do not exist is difficult to achieve.
According to one aspect, the present disclosure has an objective of making practical recommendations of changes in item values.

Solution to Problem

According to one aspect, there is provided an information processing program that causes a computer to perform the following process.
The computer obtains a plurality of attribute datasets including a subject attribute dataset, which are each a combination of a plurality of item values, and information indicating an area range that is taken as a target in a dimensional space. The computer then performs a determination process of determining a change-target item value to be changed from among the plurality of item values of the subject attribute dataset and a changing direction for the change-target item value, based on a distribution of the subject attribute dataset and at least some of the plurality of attribute datasets in the dimensional space. The computer then outputs information indicating a result of changes made by alternately iterating a changing process of changing the change-target item value by a predetermined amount or more, based on the changing direction and the determination process that follows the changing process until the subject attribute dataset is within the area range.

Advantageous Effects of Invention

According to one aspect, it is possible to make practical recommendations of changes in item values.

The foregoing and other objects, features, and advantages of the present invention will become apparent from the following description of preferred embodiments, when read together with the accompany drawings.

Brief Description of Drawings

[FIG. 1] FIG. 1 illustrates an example of a recommendation method according to a first embodiment.

[FIG. 2] FIG. 2 illustrates an example of a hardware configuration of a recommendation apparatus.

[FIG. 3] FIG. 3 is a view for describing an example of recommending an action of changing item values.

[FIG. 4] FIG. 4 illustrates an example of recommending an action.

[FIG. 5] FIG. 5 illustrates an example of recommending an action in the case where the correlation between item values is not linear.

[FIG. 6] FIG. 6 illustrates an example of recommending a first-step action that makes movement through a route where data points exist.

[FIG. 7] FIG. 7 illustrates an example of recommending a second-step action that makes movement through a route where data points exist.

[FIG. 8] FIG. 8 illustrates an example of recommending a third-step action that makes movement through a route where data points exist.

[FIG. 9] FIG. 9 is a block diagram illustrating an example of the functions of the recommendation apparatus.

[FIG. 10] FIG. 10 illustrates an example of attribute information.

[FIG. 11] FIG. 11 illustrates an example of area information.

[FIG. 12] FIG. 12 illustrates an example of setting the area information.

[FIG. 13] FIG. 13 is a flowchart illustrating an example of a procedure for an action recommendation process.

[FIG. 14] FIG. 14 is a flowchart illustrating a procedure for a movement-target data point movement process.

[FIG. 15] FIG. 15 illustrates an example of determining a destination from candidate destination points.

[FIG. 16] FIG. 16 illustrates an example of recommending an action for the first time.

[FIG. 17] FIG. 17 illustrates an example of recommending an action for the second time.

[FIG. 18] FIG. 18 illustrates an example of recommending an action for the third time.

Description of Embodiments

Embodiments will be described in detail with reference to the accompanying drawings. Some of the disclosed embodiments may be combined unless they exclude each other.

(First Embodiment)

A first embodiment will be described.

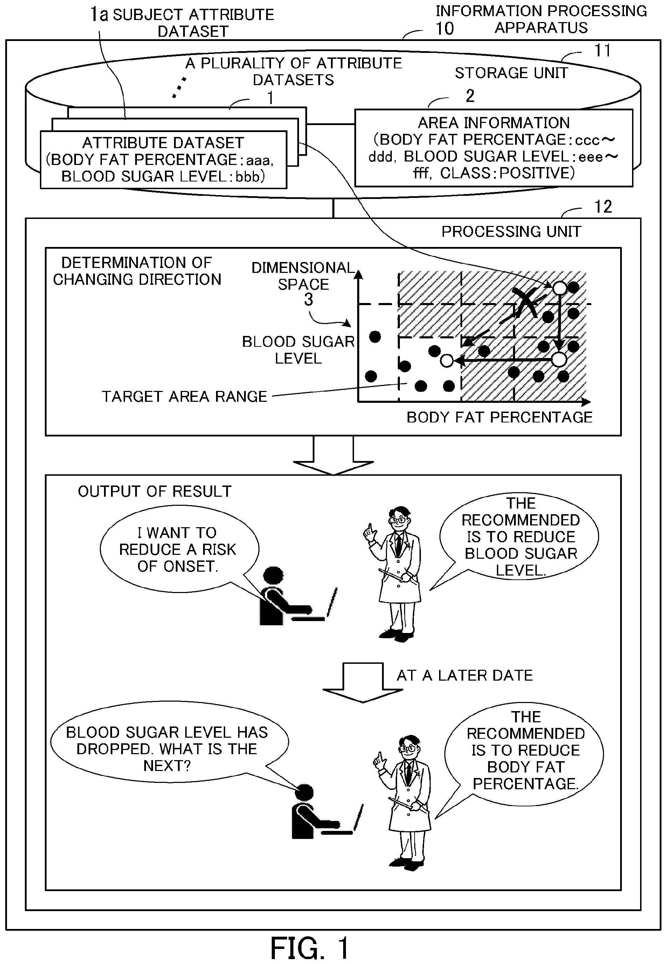

FIG. 1 illustrates an example of a recommendation method according to the first embodiment. FIG. 1 illustrates an information processing apparatus 10 that executes a recommendation method according to the first embodiment. For example, the information processing apparatus 10 is able to execute the recommendation method by running a predetermined information processing program.

The information processing apparatus 10 includes a storage unit 11 and a processing unit 12. The storage unit 11 is a memory or storage device provided in the information processing apparatus 10, for example. The processing unit 12 is a processor or computational circuit provided in the information processing apparatus 10, for example.

The storage unit 11 stores therein a plurality of attribute datasets 1 including a subject attribute dataset 1a of a subject, which are each a combination of a plurality of item values, and area information 2 indicating a target area range that is taken as a target in a dimensional space 3. For example, each of the plurality of attribute datasets 1 includes, as the item values, the values of body fat percentage and blood sugar level of a patient who has been diagnosed at a hospital or the like. In the case where an attribute dataset represents the features of a person or thing, each item value included in the attribute dataset may be called a feature quantity. The subject attribute dataset 1a is the attribute dataset of a user who wants to improve his/her health condition, for example. The target area range in the dimensional space 3 is a healthy range for body fat percentage and blood sugar level, for example. Referring to the example of FIG. 1, a range of areas that belong to a class of "positive" in the dimensional space 3 is the target area range in the dimensional space 3.

The processing unit 12 obtains the plurality of attribute datasets 1 and area information 2 from the storage unit 11. The processing unit 12 then statistically obtains the distribution of the subject attribute dataset 1a and at least some of the plurality of attribute datasets 1 in the dimensional space 3.

In this connection, at least some of the plurality of attribute datasets 1 used for obtaining the distribution in the dimensional space 3 are attribute datasets that exist within a predetermined range from the subject attribute dataset 1a, for example. The predetermined range is a range within a predetermined distance measured in Lp-norm from a point corresponding to the subject attribute dataset 1a, for example. The processing unit 12 may exclude attribute datasets that are farther from the target area range than the subject attribute dataset 1a, from the attribute datasets used for obtaining the distribution in the dimensional space 3.

Then, the processing unit 12 performs a determination process of determining a change-target item value to be changed from among the plurality of item values of the subject attribute dataset 1a and a changing direction for the change-target item value on the basis of the obtained distribution. For example, the processing unit 12 determines the change-target item value and the changing direction for the change-target item value such that the subject attribute dataset 1a approaches the area range through an area where at least some of the plurality of attribute datasets 1 are clustered in the dimensional space 3.

In addition, the processing unit 12 alternately iterates a changing process of changing the change-target item value by a predetermined amount or more on the basis of the changing direction and the determination process that follows the changing process until the subject attribute dataset 1a is within the area range. In the second and subsequent iterations of the determination process, the processing unit 12 may exclude attribute datasets existing in a direction back toward where the subject attribute dataset 1a has existed before the change, from attribute datasets used for obtaining a distribution in the dimensional space 3.

Then, the processing unit 12 outputs information indicating a result of the changes made. The output information is used for making a recommendation of changes in the item values, for example.

As described above, by iteratively determining the change-target item value and the changing direction for the change-target item value on the basis of a distribution of attribute datasets, it is possible to output information for moving the subject attribute dataset 1a through an area where attribute datasets are clustered into the target area range. Since the movement passes through the area where the attribute datasets are clustered, it is possible to make practical recommendations of changes in the item values.

For example, assume that the types of the item values are body fat percentage and blood sugar level and the values of body fat percentage and blood sugar level of the user are recognized to have a high risk of onset of diseases. When the user enters a desire to reduce the risk of onset of diseases to the information processing apparatus 10, the processing unit 12 iteratively determines the change-target item value and the changing direction for the change-target item value on the basis of a distribution of attribute datasets, using the attribute dataset of the user as the subject attribute dataset 1a.

Referring to the example of FIG. 1, the distribution of other attribute datasets around a point indicating the subject attribute dataset 1a in the dimensional space 3 is biased in a direction parallel to the axis of blood sugar level. Therefore, the processing unit 12 first determines that the change-target item value in the subject attribute dataset 1a is "blood sugar level." Here, to reduce the blood sugar level is a way to move the point corresponding to the subject attribute dataset 1a closer to the target area range by changing the value of blood sugar level. Therefore, the processing unit 12 determines that the changing direction for the change-target item value is a negative direction (a direction of decreasing the value).

The processing unit 12 changes the change-target item value "blood sugar level" of the subject attribute dataset 1a by a predetermined change amount in the direction (negative direction) determined as the changing direction. The position of the changed subject attribute dataset 1a is still outside the target area range. Therefore, the processing unit 12 determines the change-target item value and the changing direction for the change-target item value again on the basis of a distribution of other attribute datasets around the changed subject attribute dataset 1a.

Referring to the example of FIG. 1, the distribution of other attribute datasets (except for attribute datasets existing in a direction back toward the position before the change) around a point indicating the changed subject attribute dataset 1a in the dimensional space 3 is biased in a direction parallel to the axis of body fat percentage. Therefore, the processing unit 12 determines that the change-target item value in the subject attribute dataset 1a is "body fat percentage." To reduce the body fat percentage is a way to move the point corresponding to the subject attribute dataset 1a closer to the target area range by changing the value of body fat percentage. Therefore, the processing unit 12 determines that the changing direction for the change-target item value is a negative direction (a direction of decreasing the value).

The processing unit 12 changes the change-target item value "body fat percentage" of the subject attribute dataset 1a by a predetermined change amount in the direction (negative direction) determined as the changing direction. The position of the changed subject attribute dataset 1a is within the target area range. Therefore, the processing unit 12 outputs information indicating the result of the changes made.

The processing unit 12 is able to recommend actions for health improvement to the user on the basis of the output information. For example, when first receiving an input of "I want to reduce a risk of onset" from the user, the processing unit 12 recommends an action of reducing the blood sugar level by stating, "The recommended is to reduce blood sugar level." When receiving an inquiry of "Blood sugar level has dropped. What is the next?" from the user at a later date, the processing unit 12 recommends an action of reducing the body fat percentage by stating, "The recommended is to reduce body fat percentage."

The above recommendations of the actions are to change the user's health condition into a health condition with a low risk of onset through health conditions that other patients have. Therefore, these recommendations are practical.

In this connection, the processing unit 12 is able to determine the change-target item value and the changing direction for the change-target item value on the basis of the correlation relationship between the types of a plurality of item values included in attribute datasets existing within a predetermined range from the subject attribute dataset. For example, considering two types of item values, body fat percentage and blood sugar level and assuming that these two types of item values have correlation relationship, regression analysis may be performed to produce a linear equation that represents the relationship between the item values. The processing unit 12 determines the change-target item value and the changing direction for the change-target item value such as to move the point corresponding to the subject attribute dataset 1a along the gradient direction of the produced linear equation. By doing so, the processing unit 12 is able to appropriately determine the change-target item value and the changing direction for the change-target item value.

Alternatively, the processing unit 12 may determine the change-target item value and the changing direction for the change-target item value using the Mahalanobis distance. The Mahalanobis distance takes into account the correlation relationship among the types of a plurality of item values. The use of the Mahalanobis distance makes it possible to appropriately determine the change-target item value and the changing direction for the change-target item value even in the case where there are three or more types of item values.

In the case of using the Mahalanobis distance, the processing unit 12 sets a plurality of candidate destination points each of which is a predetermined distance measured in Lp-norm away from the subject attribute dataset, for example. Then, the processing unit 12 determines the change-target item value and the changing direction for the change-target item value on the basis of a candidate destination point with the minimum Mahalanobis distance from the subject attribute dataset among the plurality of candidate destination points. By doing so, the processing unit 12 is able to determine the change-target item value and the changing direction for the change-target item value such as to move the point corresponding to the subject attribute dataset 1a along a route with small Mahalanobis distance to the target area range. There are many points corresponding to other attribute datasets around the route with small Mahalanobis distance, and the movement of the subject attribute dataset 1a along such a route is feasible. As a result, it is possible to recommend definitely feasible actions to the user.

(Second Embodiment)

The following describes a second embodiment. The second embodiment relates to a recommendation apparatus that is able to make recommendations for improving user's health condition. The recommendation apparatus may be implemented by using a computer, for example.

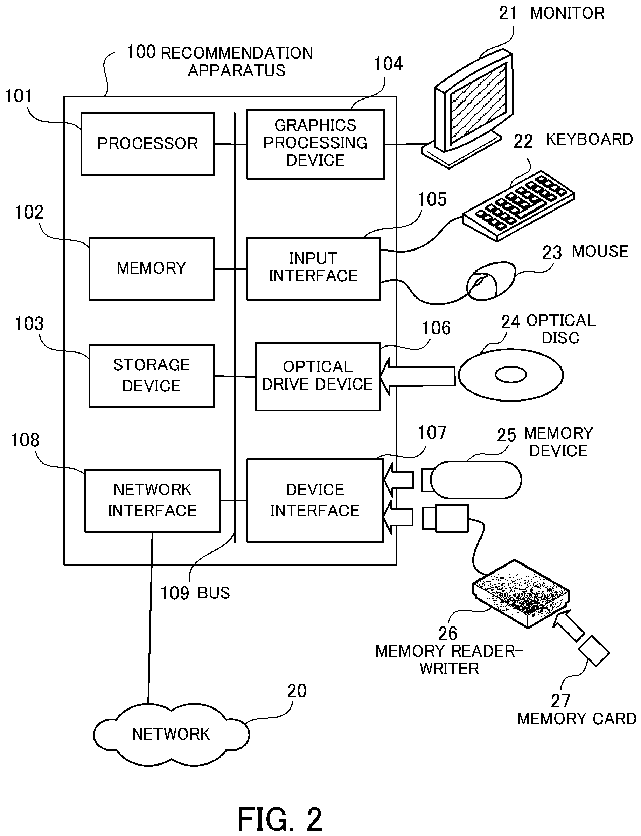

FIG. 2 illustrates an example of a hardware configuration of a recommendation apparatus. The recommendation apparatus 100 includes a processor 101 to control its entire operation. A memory 102 and a plurality of peripheral devices are connected to the processor 101 with a bus 109. The processor 101 may be a multiprocessor. For example, the processor 101 may be a central processing unit (CPU), a micro processing unit (MPU), or a digital signal processor (DSP). At least some of the functions executed by the processor 101 running programs may be implemented by using an electronic circuit such as an application specific integrated circuit (ASIC) or a programmable logic device (PLD).

The memory 102 serves as a primary storage device in the recommendation apparatus 100. The memory 102 is used to temporarily store at least part of operating system (OS) programs and application programs that the processor 101 runs. The memory 102 also stores therein various data that the processor 101 uses in its processing. For example, a random access memory (RAM) or another volatile semiconductor storage device may be used as the memory 102.

The peripheral devices connected to the bus 109 include a storage device 103, a graphics processing device 104, an input interface 105, an optical drive device 106, a device interface 107, and a network interface 108.

The storage device 103 writes and reads data electrically or magnetically on a built-in storage medium. The storage device 103 serves as a secondary storage device in a computer. The storage device 103 stores therein the OS programs, application programs, and various data. For example, a hard disk drives (HDD) or a solid state drive (SSD) may be used as the storage device 103.

A monitor 21 is connected to the graphics processing device 104. The graphics processing device 104 displays images on the monitor 21 in accordance with commands from the processor 101. The monitor 21 may be, for example, an organic electro-luminescence (EL) display device or a liquid crystal display device.

A keyboard 22 and a mouse 23 are connected to the input interface 105. The input interface 105 supplies signals from the keyboard 22 and mouse 23 to the processor 101. The mouse 23 is an example of pointing device, and another pointing device may be used. Other pointing devices include a touch panel, a tablet, a touchpad, a trackball, and others.

The optical drive device 106 performs data read and write on an optical disc 24 by using laser light or the like. The optical disc 24 is a portable storage medium on which data is recorded such as to be readable with reflection of light. The optical disc 24 may be a digital versatile disc (DVD), DVD-RAM, compact disc read-only memory (CD-ROM), CD-Recordable (CD-R), CD-Rewritable (CD-RW), or another.

The device interface 107 is a communication interface to connect peripheral devices to the recommendation apparatus 100. For example, the device interface 107 allows connections from a memory device 25 and a memory reader-writer 26. The memory device 25 is a storage medium having a capability to communicate with the device interface 107. The memory reader-writer 26 is used to perform data read and write on a memory card 27. The memory card 27 is a card-type storage medium.

The network interface 108 is connected to a network 20. The network interface 108 communicates data with other computers and communication devices over the network 20. For example, the network interface 108 is a wired communication interface that is connected to a wired communication device such as a switch or a router with a cable. Alternatively, the network interface 108 may be a wireless communication interface that is connected to a wireless communication device such as a base station or an access point with radio waves.

The recommendation apparatus 100 with the above hardware configuration is able to implement the processing functions of the second embodiment. In this connection, the information processing apparatus 10 according to the first embodiment may be configured with the same hardware as the recommendation apparatus 100 illustrated in FIG. 2.

The recommendation apparatus 100 implements the processing functions of the second embodiment by, for example, running programs stored in a computer-readable storage medium. The programs describing the processing functions to be executed by the recommendation apparatus 100 may be stored in a variety of storage media. For example, the programs to be run by the recommendation apparatus 100 may be stored in the storage device 103. The processor 101 loads at least part of a program from the storage device 103 into the memory 102 and runs the loaded program. The programs to be run by the recommendation apparatus 100 may be stored in the optical disc 24, memory device 25, memory card 27, or another portable storage medium. The programs stored in such a portable storage medium are installed in the storage device 103 under the control of the processor 101, so that they are ready to execute. In addition, the processor 101 is able to read the programs directly from the portable storage medium and run the read programs.

For example, for a person who is currently expected to be unhealthy, the recommendation apparatus 100 is able to determine how to change his/her health condition so that he/she will be expected to be healthy, and makes an improvement recommendation for him/her to be healthy. The improvement recommendation includes an improvement action of changing an appropriate item value for improving health among a plurality of item values (blood sugar level, body fat percentage, and others) relating to health conditions. In recommending the improvement action, the recommendation apparatus 100 avoids making an unrealistic recommendation, taking into account a distribution of datasets indicating many other people's health conditions.

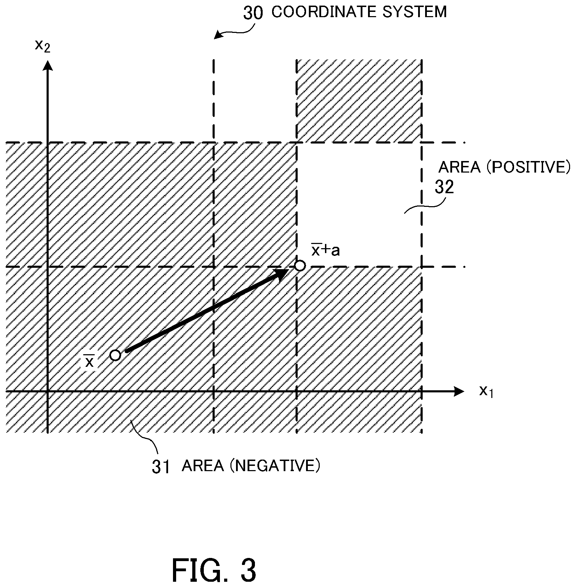

The following describes an example of making a recommendation that is not feasible, with reference to FIGS. 3 to 5.

FIG. 3 is a view for describing an example of recommending an action of changing item values. For example, assume that an input x (with overline) with a plurality of item values regarding user's health condition is entered to a model H that predicts the user's health condition. Various models may be used as the model of obtaining a predicted value from input item values. For example, a generalized additive model (GAM) may be used. Alternatively, a linear classification model may be used. Linear classification models include logistic regression models, linear support vector machines, and others. Yet alternatively, a decision-tree ensemble model may be used. A decision tree represents a set of prediction rules in the form of a binary tree. Decision-tree ensemble models include random forests, gradient boosted trees (XGBoost or the like), ExtraTrees, and others. Yet alternatively, a generalized linear model may be used. In the example of FIG. 3, it is assumed that item values $x_1$ and $x_2$ regarding health are set for the input x (with overline).

The recommendation apparatus 100 is able to predict a class in response to an input using a model. For example, the model predicts a class indicating the presence or absence of a health-related risk. For example, the model predicts whether a risk of onset of a predetermined disease is high or low. FIG. 3 illustrates a coordinate system 30 with the horizontal axis representing the item value $x_1$ and the vertical axis representing the item value $x_2$. The coordinate system 30 is divided into an area 31 corresponding to a negative class (with a high risk of onset) and an area 32 corresponding to a positive class (with a low risk of onset). Referring to FIG. 3, the area 31 corresponding to the negative class is hatched.

Assume now that a data point corresponding to user's current values of the item values $x_1$ and $x_2$ exists in the area 31 corresponding to the negative class in the coordinate system 30. In such a case, an improvement action a* of changing the values of the item values so that the prediction result obtained by the model H belongs to the other class is represented by equation (1).

$$a^* = argmin_a C(a \mid \overline{x}) s.t. H(\overline{x}) \neq H(\overline{x} + a) \qquad (1)$$

The improvement action a* is a set of vector data (action a) that has change amounts of the item value $x_1$ and item value $x_2$ as components. In equation (1), "C(a|x)" (x with overline) denotes a cost function and is, for example, a weighted Lp-norm (p is a real number of 0 or greater). The Lp-norm denotes distance in the coordinate space. $L_2$-norm is Euclidean distance. "argmin" denotes an argument that determines the minimum value. From equation (1), the set of the action a is obtained which has the minimum distance between the start point and the end point in order to change the class of the prediction result (H(x) ≠ H(x+a)) (x with overline).

For example, assume that, with equation (1), a prediction result of the model H(x) (x with overline) belongs to the negative class. At this time, an action a is obtained which indicates a route with the minimum distance so that the prediction result of the model H(x+a) (x with overline) belongs to the positive class. This action a meets the user's desire to change the predicted value with the least effort.

The use of the distance as the cost function as described above enables changing a class with the minimum effort. For example, an action a that changes the class with a high risk of onset to the class with a low risk of onset with the minimum effort is recommended to the user. However, the action a with the minimum distance may actually be difficult to achieve. For example, a recommendation of an action of increasing muscle mass and losing weight at the same time is considered. Even when such an action is recommended, it may be difficult to achieve both the muscle increase and weight loss at the same time, because muscle is heavier than fat and the replacement of fat with muscle increases weight.

In addition, the coordinate system 30 in the model may have an area which corresponds to the positive class but to which no people actually belong or in which existence of such people is rare. In this case, an action that makes movement to such an area, even recommended, is difficult to achieve.

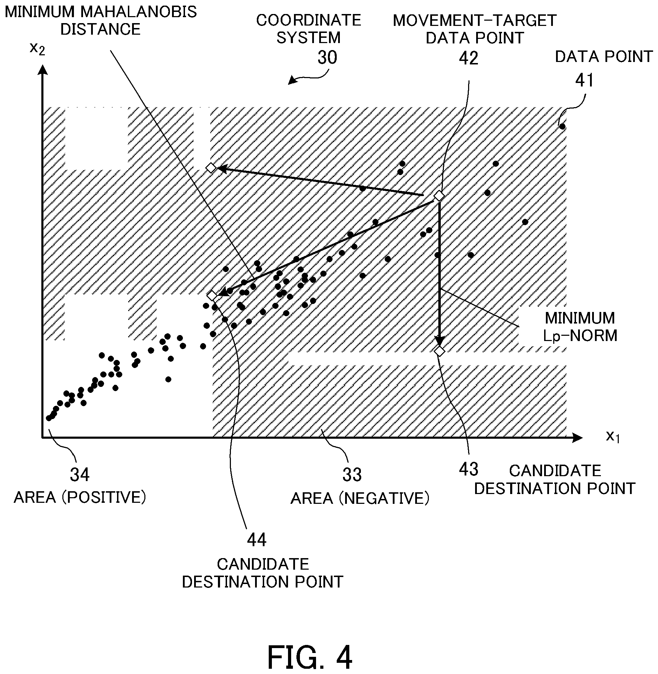

FIG. 4 illustrates an example of recommending an action. Referring to FIG. 4, data points 41 corresponding to combinations of item values regarding many people's health are plotted in the coordinate system 30. Here, a data point corresponding to user's combination of item values whose class, indicated by a prediction result of the model, is to be changed is a movement-target data point 42 to be moved.

The movement-target data point 42 exists in an area 33 corresponding to the negative class. Assume the case of recommending an action that moves the movement-target data point 42 to an area 34 corresponding to the positive class. In this case, a recommendation of an action that makes movement to a candidate destination point 43 with the minimum distance in the Lp-norm is a recommendation of movement to a location where no other data points exist. The location where no other data points exist corresponds to a health condition that no person has, and a recommendation of an action of becoming such a health condition is improper.

It is now considered to recommend an action that moves the movement-target data point 42, taking into account the correlation between the item values. To take into account the correlation between the item values, the Mahalanobis distance (distance taking correlation into account) is usable as a cost function, for example. Let D be the number of types of item values. The Mahalanobis distance $d_M(x, y)$ for vectors x, y ∈ $R^D$ (R is a set of real numbers) following the same distribution is defined by equation (2).

$$d_M(x, y) = \sqrt{(y - x)^T \Sigma^{-1} (y - x)} \qquad (2)$$

In equation (2), $\Sigma \in R^{D \times D}$ is a covariance matrix. In the case where the item values have correlation with each other, a straight line that is generated by regression analysis to represent the relationship between the item values has the following feature: a point around the straight line and a point distant from the straight line may have the same Lp-norm but have different Mahalanobis distances. An area close to the straight line representing the relationship between the item values has small Mahalanobis distances, and an area distant from the straight line has large Mahalanobis distances.

The Mahalanobis distance is not solvable with a realistic time frame with the strict linearization using a planning technique. Therefore, the recommendation apparatus 100 introduces a surrogate function based on an eigenvalue decomposition of the covariance matrix to approximately obtain the Mahalanobis distance. More concretely, the surrogate function that approximates the Mahalanobis distance $d_M(x, y)$ using the eigenvalues and eigenvectors of the covariance matrix $\Sigma$ is defined. In this connection, when the number of types of item values is D, D eigenvalues and D eigenvectors of the covariance matrix $\Sigma$ are obtained. The surrogate function is represented by equation (3) where $\lambda_d$ denotes the d-th eigenvalue (d is an integer of 1 to D) and $u_d$ denotes the d-th eigenvector.

$$\hat{d}_M(x,y) := \sum_{d=1}^{D} \sqrt{\lambda_d^{-1}|(y-x)^T u_d|} \qquad (3)$$

In equation (3), $\Sigma$ denotes a summation. The square root part in equation (3) calculates a pseudo Mahalanobis distance. Since D denotes the number of types of item values, the sum of pseudo Mahalanobis distances obtained for all types of item values is an approximate Mahalanobis distance.

In the case where the correlation between item values is linear (linear correlation), a recommendation of an action that makes movement to a candidate destination point 44 with the minimum Mahalanobis distance is equivalent to a recommendation of an action that makes movement through a route where other data points exist. In the case where the correlation between the item values is not linear, however, an action that makes movement through a route where other data points do not exist may be recommended, even when the Mahalanobis distance is used.

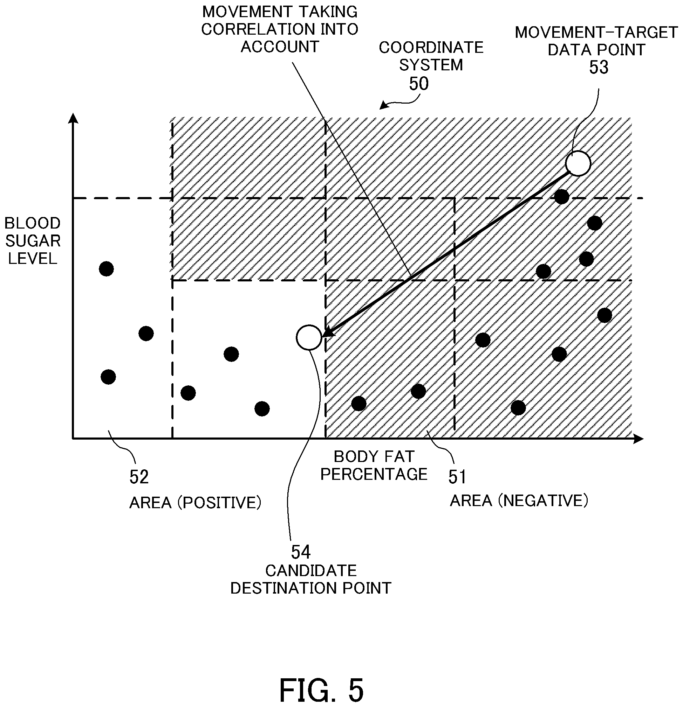

FIG. 5 illustrates an example of recommending an action in the case where the correlation between item values is not linear. A coordinate system 50 illustrated in FIG. 5 has a horizontal axis representing body fat percentage and a vertical axis representing blood sugar level. Data points corresponding to many patients' body fat percentage and blood sugar level are plotted in the coordinate system 50. The body fat percentage and blood sugar level do not have a linear correlation with each other but have relationship similar to a second-order correlation.

Consider now the case of recommending an action that moves a movement-target data point 53 existing in an area 51 corresponding to a negative class to an area 52 corresponding to a positive class. In the example of FIG. 5, the correlation between the different types of item values is not linear. If, using all plotted points and assuming that the item values have a linear correlation with each other, an action specifying a candidate destination point 54 with the minimum Mahalanobis distance as a destination is recommended, this results in recommending the action that makes movement through a route where no data points exist.

As described now, in the case of searching for a destination to be recommended, simply taking into account the correlation, there is a possibility that the recommendation is not feasible. To deal with this, the recommendation apparatus 100 recommends actions stepwise. More specifically, the recommendation apparatus 100 makes stepwise action recommendations so that the plurality of actions result in movement to an area corresponding to a target class. At this time, the recommendation apparatus 100 recommends the actions that make movement through a route where data points exist, taking into account the correlation between the item values for each action. To this end, the recommendation apparatus 100 determines the position of a destination using only the correlation around the current position.

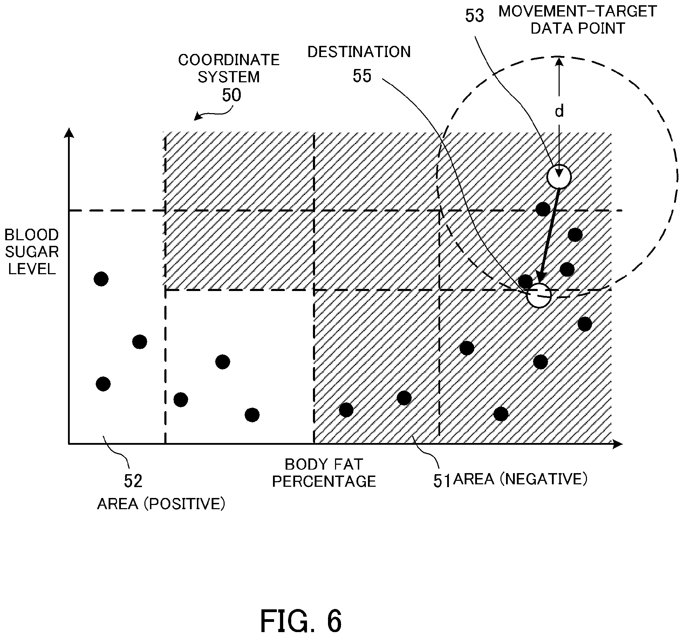

FIG. 6 illustrates an example of recommending a first-step action that makes movement through a route where data points exist. The recommendation apparatus 100 obtains the correlation between the item values only from data points around the movement-target data point 53. The data points around the movement-target data point 53 are data points existing within a distance d in the Euclidean distance from the movement-target data point 53, for example. Then, the recommendation apparatus 100 recommends the first-step action that specifies a destination 55 that is, for example, the distance d in the Euclidean distance away from the movement-target data point 53 on a straight line representing the correlation. The destination 55 is a position located in a direction approaching the area 52 corresponding to the positive class from the movement-target data point 53.

Then, the recommendation apparatus 100 moves the movement-target data point 53 to the position of the destination 55 and determines a destination for recommending a second-step action.

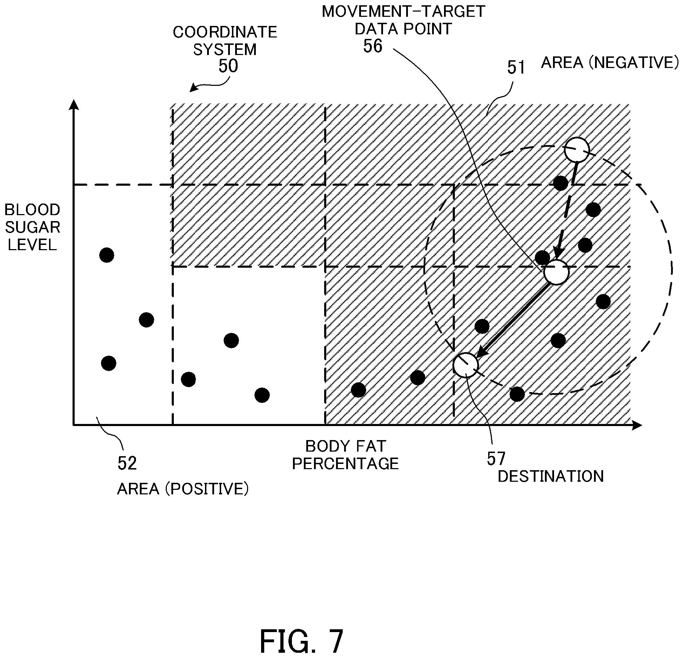

FIG. 7 illustrates an example of recommending the second-step action that makes movement through a route where data points exist. The recommendation apparatus 100 obtains the correlation between the item values only from data points around the movement-target data point 56. At this time, the recommendation apparatus 100 may obtain the correlation without taking into account data points existing in the direction back toward the moving source position. The data points in the direction back toward the moving source position are data points existing within the distance d from the movement-target data point 53 (FIG. 6) before the movement. The recommendation apparatus 100 obtains the correlation between the different types of item values on the basis of the data points that exist within the distance d from the movement-target data point 56 and are the distance d or more away from the movement-target data point 53 before the movement, for example.

Then, the recommendation apparatus 100 recommends the second-step action that specifies a destination 57 that is, for example, the distance d in the Euclidean distance away from the movement-target data point 56 on a straight line representing the correlation. The destination 57 is a position located in a direction approaching the area 52 corresponding to the positive class from the movement-target data point 56.

After that, the recommendation apparatus 100 moves the movement-target data point 56 to the position of the destination 57 and determines a destination for recommending a third-step action.

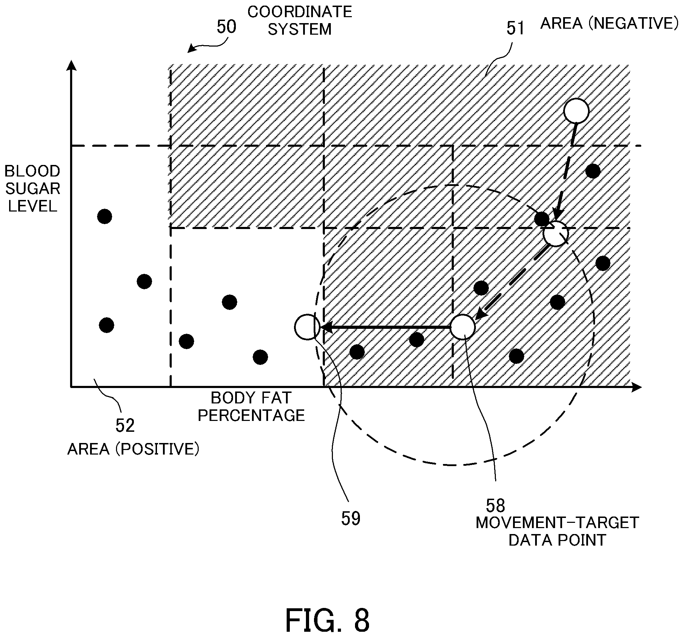

FIG. 8 illustrates an example of recommending the third-step action that makes movement through a route where data points exist. The recommendation apparatus 100 obtains the correlation between the item values only from data points around the movement-target data point 58. At this time, the recommendation apparatus 100 may obtain the correlation without taking into account data points existing in the direction back toward the moving source position. Then, the recommendation apparatus 100 recommends the third-step action that specifies a destination 59 that is, for example, the distance d in the Euclidian distance away from the movement-target data point 58 on a straight line representing the correlation. The destination 59 is a position located in a direction approaching the area 52 corresponding to the positive class from the movement-target data point 56.

Referring to the example of FIG. 8, the destination 59 is within the area 52 corresponding to the positive class. Therefore, the recommendation of the three, first-step to third-step, actions is a recommendation of actions to make the user with the health condition represented by the original movement-target data point 53 have a positive health condition. Note that each recommended action is a feasible action because the movement passes through the areas where data points exist.

The following describes the functions of the recommendation apparatus 100 for recommending feasible actions.

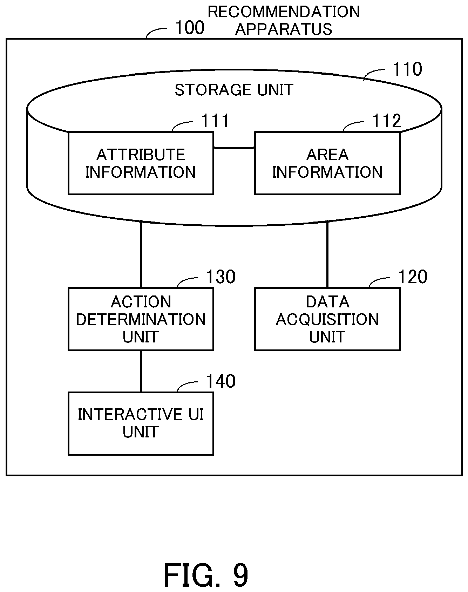

FIG. 9 is a block diagram illustrating an example of the functions of the recommendation apparatus. The recommendation apparatus 100 includes a storage unit 110, a data acquisition unit 120, an action determination unit 130, and an interactive user interface (UI) unit 140.

The storage unit 110 stores therein attribute information 111 and area information 112. The attribute information 111 is sample data of item values regarding the health conditions of many people. For example, the sample data indicates body fat percentage, blood sugar level, and others. The area information 112 is data indicating classes set for areas in a coordinate system that has a coordinate for each type of item values. The classes include a positive class and a negative class, for example. The storage unit 110 may be implemented by using part of the storage space of the memory 102 or storage device 103 provided in the recommendation apparatus 100, for example.

The data acquisition unit 120 obtains the attribute information 111 and area information 112. For example, the data acquisition unit 120 receives an input of the attribute information 111 and stores the received attribute information 111 in the storage unit 110. In addition, the data acquisition unit 120 receives an input of input data specifying the range and class of an area and stores the input data as a record of the area information 112 in the storage unit 110.

The action determination unit 130 determines an action to be recommended to a user. For example, the action determination unit 130 recommends one or more actions that need to be taken in time series to change the class of the area to which a data point corresponding to the user's current item values belongs in the coordinate system, taking into account the correlation between the item values indicated in the attribute information 111.

The interactive UI unit 140 outputs the recommendations of the actions. For example, the interactive UI unit 140 performs voice interaction with the user via an artificial intelligence (AI) assistant. The interactive UI unit 140 outputs a voice message for presenting the content of an action recommended for improving a health condition in response to an input of user's voice for an inquiry about a plan for improving the health condition.

By operating the above functional elements in conjunction with each other, the recommendation apparatus 100 is able to recommend, to the user, an action plan for changing the user's health condition to his/her desired health condition. The functions of the elements illustrated in FIG. 9 may be implemented by a computer executing the program modules corresponding to the elements, for example.

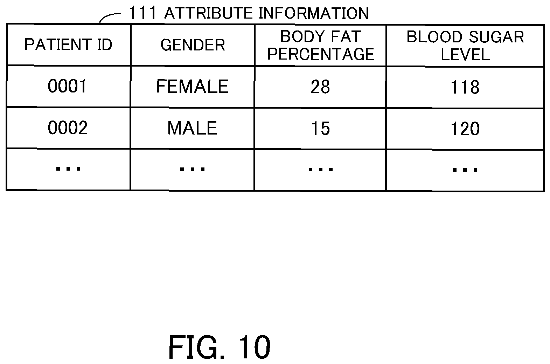

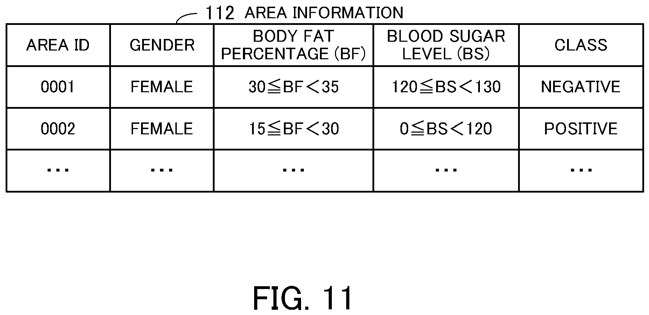

The following describes data stored in the storage unit 110 concretely with reference to FIGS. 10 and 11.

FIG. 10 illustrates an example of attribute information. The attribute information 111 includes a plurality of records containing item values regarding patients' health conditions. Each record in the attribute information 111 is an example of the attribute datasets used in the first embodiment. Each record in the attribute information 111 has the following fields: patient ID, gender, body fat percentage, and blood sugar level. The patient ID field contains the identifier (patient ID) of a patient. The gender field contains the gender of the patient. The body fat percentage field contains the body fat percentage of the patient, and the blood sugar level field contains the blood sugar level of the patient.

In the attribute information 111 of FIG. 10, the item values indicating the patients' health conditions are body fat percentage and blood sugar level. In this connection, a proper range for an item value indicating a health condition may depend on gender. For example, a proper range of body fat percentage for males is lower than that for females. For this reason, it is proper to set a class depending on gender for each area in a coordinate system having axes respectively for the types of item values.

FIG. 11 illustrates an example of area information. The area information 112 includes a plurality of records specifying the classes of areas. Each record includes the following fields: area ID, gender, body fat percentage (BF), blood sugar level (BS), and class. The area ID field contains the identifier (area ID) of an area. The gender field indicates whether the area relates to a class for males or for females. The body fat percentage field contains a range of body fat percentage in the area. The blood sugar level field contains a range of blood sugar level in the area. The class field contains the name of the class corresponding to the area.

The following describes an example of setting the area information. For example, the user enters information specifying an area within the coordinate system 50 and information specifying a class to the recommendation apparatus 100.

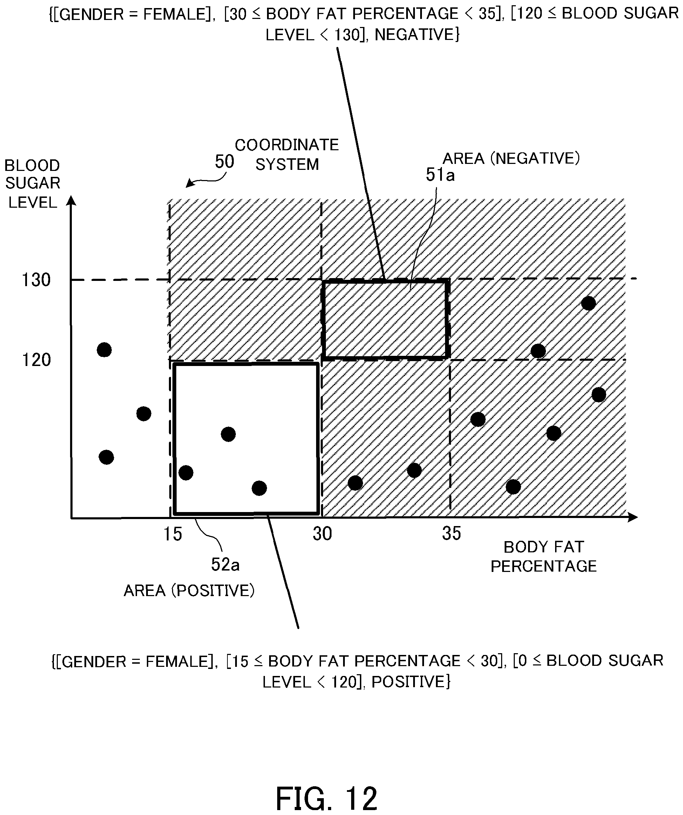

FIG. 12 illustrates an example of setting the area information. For example, when the user enters "{[gender = female], [30 ≤ body fat percentage < 35], [120 ≤ blood sugar level < 130], negative}," a class of negative is set for the corresponding area 51a in the coordinate system 50. When the user enters "{[gender = female], [15 ≤ body fat percentage < 30], [0 ≤ blood sugar level < 120], positive}," a class of positive is set for the corresponding area 52a in the coordinate system 50.

The user sets a class for each area in the entire coordinate system 50. Then, the user specifies one data point in a negative area and causes the recommendation apparatus 100 to recommend an action that moves the data to a positive area.

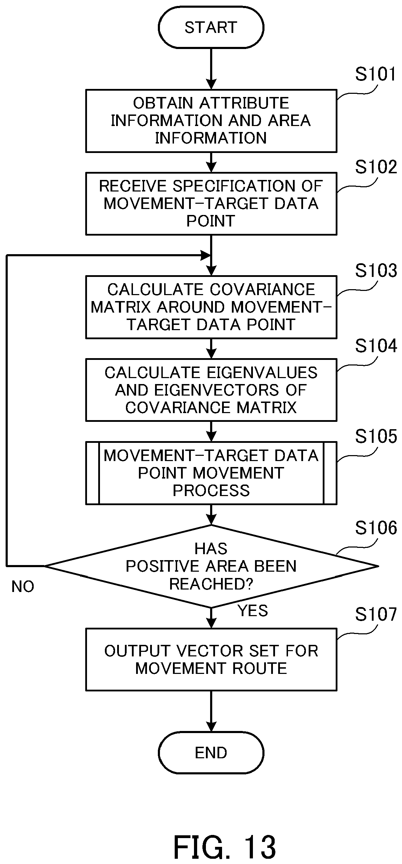

FIG. 13 is a flowchart illustrating an example of a procedure for an action recommendation process. In the following, the process of FIG. 13 will be described step by step.

(Step S101) The data acquisition unit 120 obtains attribute information 111 and area information 112. For example, the data acquisition unit 120 receives an input specifying patient electronic health record data from a user, extracts the attribute information 111 from the electronic health record data, and stores the extracted attribute information 111 in the storage unit 110. In addition, the data acquisition unit 120 receives input data for setting a class for each area as illustrated in FIG. 12, and adds a record corresponding to the input data to the area information 112 in the storage unit 110.

(Step S102) The interactive UI unit 140 receives, from the user, an input specifying a movement-target data point to be moved, which is set in an area corresponding to a negative class. For example, the interactive UI unit 140 receives an input specifying a patient ID and takes a data point corresponding to the body fat percentage and blood sugar level associated with the patient ID as the movement-target data point.

(Step S103) The action determination unit 130 obtains the attribute information 111 from the storage unit 110, and calculates a covariance matrix based on data points around the movement-target data point (for example, within a predetermined value in Lp-norm) on the basis of the attribute information 111. The covariance matrix is a multi-dimensional extension of the concept of variance for different types of item values. The covariance matrix reflects the strength of the correlation between the types of item values.

(Step S104) The action determination unit 130 calculates the eigenvalues $\lambda$ and eigenvectors u of the covariance matrix. When the number of types of item values in the attribute information 111 is D, D combinations of eigenvalue $\lambda$ and eigenvector u obtained for the eigenvalue $\lambda$ are generated. The D combinations of eigenvalue $\lambda$ and eigenvector u are used for calculating a Mahalanobis distance.

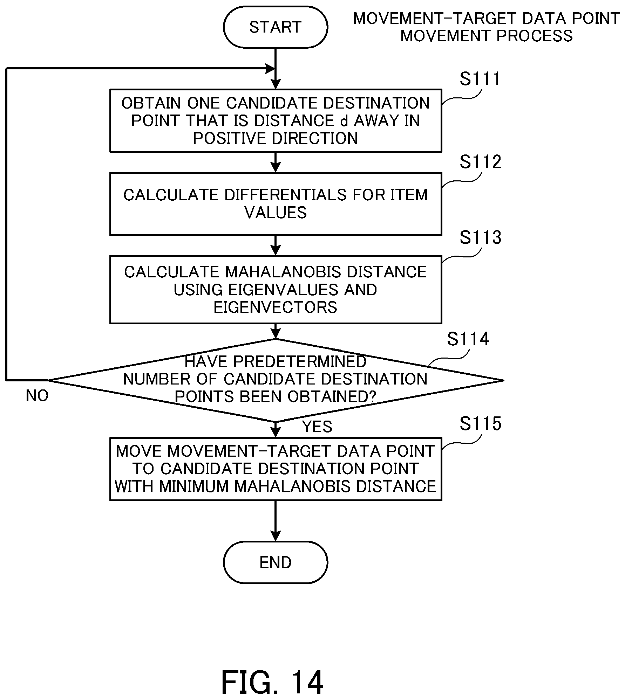

(Step S105) The action determination unit 130 performs a movement-target data point movement process. This process is to move the movement-target data point by a fixed distance d through an area around which other data points exist, in a direction approaching a positive area. This movement-target data point movement process will be described in detail later (with reference to FIG. 14).

(Step S106) The action determination unit 130 determines whether the movement-target data point has reached the positive area. For example, the action determination unit 130 obtains the area information 112 from the storage unit 110. Then, the action determination unit 130 detects the class of an area that has the position of the movement-target data point moved through the movement-target data point movement process, on the basis of the obtained area information 112. The action determination unit 130 determines that the movement-target data point has reached the positive area when the class of the area is positive. If the movement-target data point has reached the positive area, the process proceeds to step S107. If the movement-target data point has not reached the positive area, the process proceeds to step S103.

(Step S107) The action determination unit 130 outputs a vector set of vectors for a movement route determined for the movement-target data point. For example, the action determination unit 130 sends the vector set for the movement route to the interactive UI unit 140. The interactive UI unit 140 recommends actions to the user on the basis of the directions and others of vectors included in the vector set for the movement route.

The following describes the movement-target data point movement process.

FIG. 14 is a flowchart illustrating a procedure for the movement-target data point movement process. The process of FIG. 14 will be described step by step.

(Step Sill) The action determination unit 130 obtains one candidate destination point that is a distance d in Lp-norm (for example, in Euclidian distance) away in a positive direction. For example, the action determination unit 130 obtains a point that is closer to the positive area than the current position and that has not been obtained as a candidate destination point among points positioned at predetermined intervals on a predetermined circle with a radius of d from the movement-target data point.

(Step S112) The action determination unit 130 calculates the differential between the movement-target data point and the candidate destination data point for each type of item values. For example, assuming that a vector indicating the movement-target data point is $x(x_1, x_2)$ and a vector representing the candidate destination data point is $y(y_1, y_2)$, the action determination unit 130 calculates "$y_1 - x_1$" and "$y_2 - x_2$."

(Step S113) The action determination unit 130 calculates a Mahalanobis distance using the eigenvalues and eigenvectors obtained from the covariance matrix. For example, with respect to each combination of an eigenvalue and an eigenvector, the action determination unit 130 multiplies by the eigenvalue the absolute value of the product (inner product) of the vector with the differential values for respective types of item values calculated at step S112 and the eigenvector, and calculates the square root of the multiplication result. By doing so, the action determination unit 130 obtains D pseudo Mahalanobis distances. The action determination unit 130 then calculates the sum of the obtained pseudo Mahalanobis distances as the Mahalanobis distance.

(Step S114) The action determination unit 130 determines whether a predetermined number of candidate destination points have been obtained. If the action determination unit 130 has obtained the predetermined number of candidate destination points, the process proceeds to step S115. If the action determination unit 130 has not obtained the predetermined number of candidate destination points, the process proceeds to step Sill.

(Step S115) The action determination unit 130 determines that the position of a candidate destination point with the minimum Mahalanobis distance is a destination for the movement-target data point. Then, the action determination unit 130 moves the movement-target data point to the position of the determined destination. At this time, the action determination unit 130 stores a vector from the moving source position to the position of the destination of the movement-target data point as data for recommending an action in the memory 102 or storage device 103.

As described above, it is possible to move the movement-target data point such as to decrease the Mahalanobis distance.

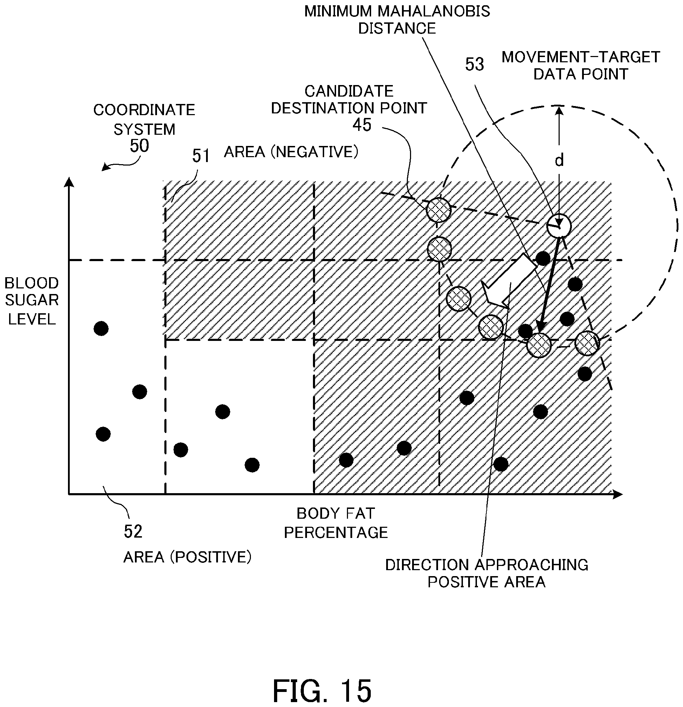

FIG. 15 illustrates an example of determining a destination from candidate destination points. As illustrated in FIG. 15, a plurality of candidate destination points 45 are set on a circle of radius d with the movement-target data point 53 as the center. Only candidate destination points 45 positioned in a direction approaching the positive area 52 are set. This avoids recommending an action that moves the movement-target data point 53 away from the positive area.

The plurality of candidate destination points 45 have an equal distance in Lp-norm from the movement-target data point 53, but have different Mahalanobis distances. Candidate destination points that do not have other data points around their routes leading to the movement-target data point 53 have large Mahalanobis distances. Candidate destination points that have more other data points around their routes leading to the movement-target data point 53 have smaller Mahalanobis distances. The action determination unit 130 selects a candidate destination point with the minimum Mahalanobis distance as a destination. This avoids recommending an action that makes movement through a route where no other data points exist.

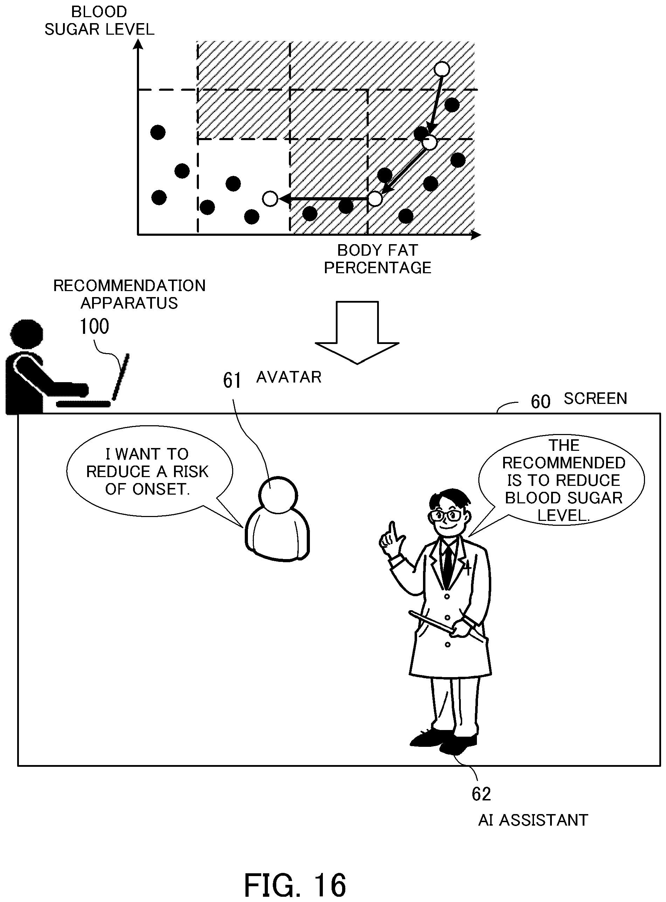

On the basis of data indicating each movement of the movement-target data point to the positive area, determined by the action determination unit 130, the interactive UI unit 140 recommends actions to the user. The following describes an example of recommending actions with reference to FIGS. 16 to 18.

FIG. 16 illustrates an example of recommending an action for the first time. Referring to the example of FIG. 16, it is assumed that a data point corresponding to the body fat percentage and blood sugar level of a user is already specified as a movement-target data point. At this time, for example, the user inputs a voice saying "I want to reduce a risk of onset."

A user avatar 61 and an AI assistant 62 are displayed on the screen 60 of the recommendation apparatus 100. The text of the user's voice is displayed in a speech bubble of the avatar 61.

The recommendation apparatus 100 makes an improvement recommendation to move the data point corresponding to the user's body fat percentage and blood sugar level to a positive area via one or more steps. Referring to the example of FIG. 16, the destination from the current position of the movement-target data point, which is determined by the action recommendation process, is a position with lower blood sugar level and almost the same body fat percentage. In the case where the value of a component of a vector representing a movement of the movement-target data point is less than or equal to a predetermined value, the interactive UI unit 140 regards the value of the component as zero and recommends an action. In this case, the interactive UI unit 140 displays a message of "The recommended is to reduce blood sugar level" in a speech bubble of the AI assistant 62.

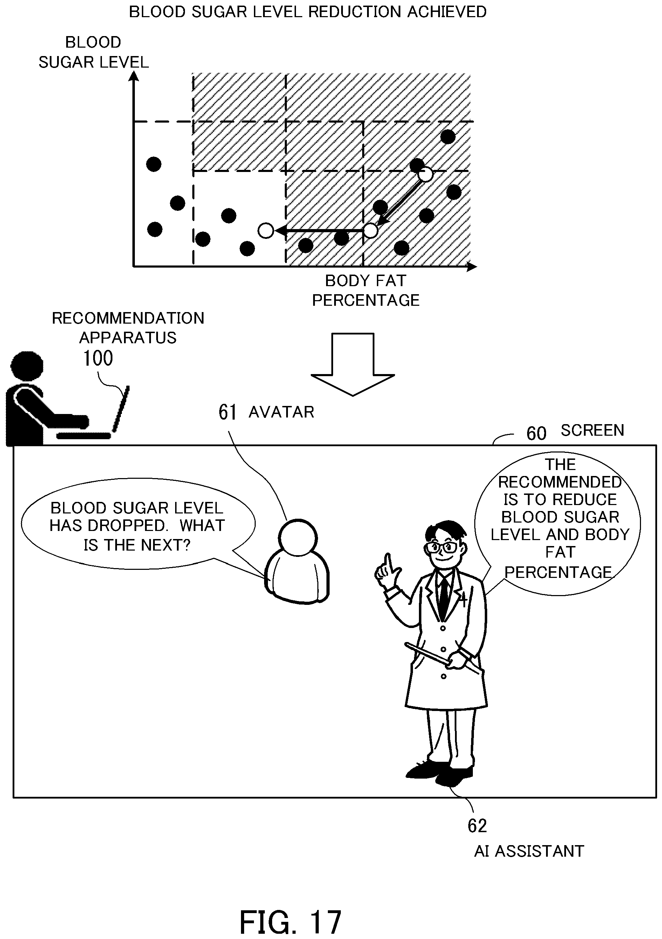

FIG. 17 illustrates an example of recommending an action for the second time. In the example of FIG. 17, it is assumed that the user has reduced his/her blood sugar level in accordance with the recommended action and a data point corresponding to the most recent body fat percentage and blood sugar level is specified as the movement-target data point. At this time, for example, the user inputs a voice saying "Blood sugar level has dropped. What is the next?" The input content is displayed in a speech bubble of the avatar 61.

The recommendation apparatus 100 makes an improvement recommendation to move the data point corresponding to the user's body fat percentage and blood sugar level to the positive area via one or more steps. Referring to the example of FIG. 17, a destination from the current position of the movement-target data point, which is determined by the action recommendation process, is a position with both lower body fat percentage and lower blood sugar level. Therefore, the interactive UI unit 140 displays a message of "The recommended is to reduce blood sugar level and body fat percentage" in a speech bubble of the AI assistant 62.

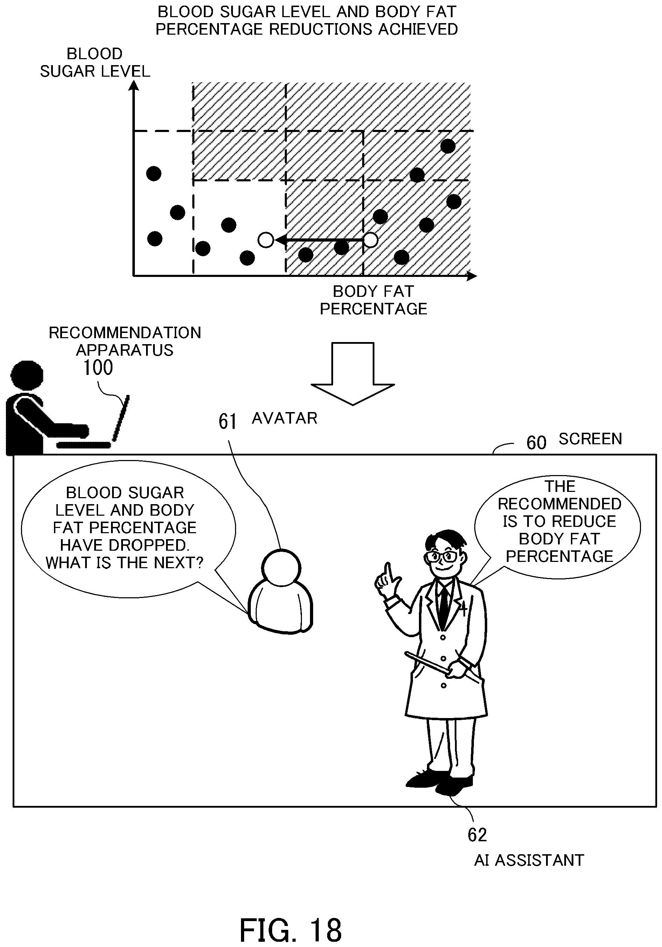

FIG. 18 illustrates an example of recommending an action for the third time. In the example of FIG. 18, it is assumed that the user has reduced his/her body fat percentage and blood sugar level in accordance with the recommended action and a data point corresponding to the most recent body fat percentage and blood sugar level is specified as the movement-target data point. At this time, for example, the user inputs a voice saying "Blood sugar level and body fat percentage have dropped. What is the next?" The input content is displayed in a speech bubble of the avatar 61.

The recommendation apparatus 100 makes an improvement recommendation to move the data point corresponding to the user's body fat percentage and blood sugar level to the positive area via one or more steps. Referring to FIG. 18, a destination from the current position of the movement-target data point, which is determined by the action recommendation process, is a position with lower body fat percentage together. Therefore, the interactive UI unit 140 displays a message of "The recommended is to reduce body fat percentage" in a speech bubble of the AI assistant 62.

As described above, an action to be recommended is determined with taking correlation into account and on the basis of a distribution of data points. By doing so, it is possible to recommend the action that is feasible for the user.

(Other Embodiments)

The second embodiment describes making an action recommendation to recommend only the next action to a user. Alternatively, the recommendation apparatus 100 is able to collectively recommend all actions for the user to have a positive condition.

The above description is merely indicative of the principles of the present embodiments. A wide variety of modifications and changes may also be made by those skilled in the art. The present embodiments are not limited to the precise configurations and example applications indicated and described above, and all appropriate modifications and equivalents are regarded as falling within the scope of the embodiments as defined by the appended patent claims and their equivalents.

Reference Signs List

1 A plurality of attribute datasets
1a Subject attribute dataset
2 Area information
3 Dimensional space
10 Information processing apparatus
11 Storage unit
12 Processing unit

**Claims**

1. An information processing program that causes a computer to perform a process comprising:

    obtaining a plurality of attribute datasets including a subject attribute dataset and information indicating an area range that is taken as a target in a dimensional space, the plurality of attribute datasets each being a combination of a plurality of item values;
    performing a determination process of determining a change-target item value to be changed from among the plurality of item values of the subject attribute dataset and a changing direction for the change-target item value, based on a distribution of the subject attribute dataset and at least some of the plurality of attribute dataset in the dimensional space; and
    outputting information indicating a result of changes made by alternately iterating a changing process of changing the change-target item value by a predetermined amount or more, based on the changing direction and the determination process that follows the changing process until the subject attribute dataset is within the area range.

2. The information processing program according to claim 1, wherein the change-target item value and the changing direction for the change-target item value are determined such that the subject attribute dataset approaches the area range through an area where at least some of the plurality of attribute datasets are clustered in the dimensional space.

3. The information processing program according to claim 1 or 2, wherein the change-target item value and the changing direction for the change-target item value are determined based on a distribution of attribute datasets existing within a predetermined range from the subject attribute dataset in the dimensional space.

4. The information processing program according to claim 3, wherein the change-target item value and the changing direction for the change-target item value are determined based on correlation relationship between types of the plurality of item values included in the attribute datasets existing within the predetermined range from the subject attribute dataset.

5. The information processing program according to claim 4, wherein the change-target item value and the changing direction for the change-target item value are determined based on a candidate destination point with minimum Mahalanobis distance from the subject attribute dataset among a plurality of candidate destination points with a predetermined distance in Lp-norm from the subject attribute dataset.

6. A recommendation method executed by a computer, the recommendation method comprising:

   obtaining a plurality of attribute datasets including a subject attribute dataset and information indicating an area range that is taken as a target in a dimensional space, the plurality of attribute datasets each being a combination of a plurality of item values;
   performing a determination process of determining a change-target item value to be changed from among the plurality of item values of the subject attribute dataset and a changing direction for the change-target item value, based on a distribution of the subject attribute dataset and at least some of the plurality of attribute dataset in the dimensional space; and
   outputting information indicating a result of changes made by alternately iterating a changing process of changing the change-target item value by a predetermined amount or more, based on the changing direction and the determination process that follows the changing process until the subject attribute dataset is within the area range.

7. An information processing apparatus comprising:
   a processing unit that performs a process of

   obtaining a plurality of attribute datasets including a subject attribute dataset and information indicating an area range that is taken as a target in a dimensional space, the plurality of attribute datasets each being a combination of a plurality of item values,
   performing a determination process of determining a change-target item value to be changed from among the plurality of item values of the subject attribute dataset and a changing direction for the change-target item value, based on a distribution of the subject attribute dataset and at least some of the plurality of attribute datasets in the dimensional space, and
   outputting information indicating a result of changes made by alternately iterating a changing process of changing the change-target item value by a predetermined amount or more, based on the changing direction and the determination process that follows the changing process until the subject attribute dataset is within the area range.

FIG. 1

100 RECOMMENDATION APPARATUS

101 PROCESSOR

104 GRAPHICS PROCESSING DEVICE

21 MONITOR

22 KEYBOARD

102 MEMORY

105 INPUT INTERFACE

23 MOUSE

103 STORAGE DEVICE

106 OPTICAL DRIVE DEVICE

24 OPTICAL DISC

108 NETWORK INTERFACE

107 DEVICE INTERFACE

25 MEMORY DEVICE

109 BUS

26 MEMORY READER-WRITER

27 MEMORY CARD

20 NETWORK

FIG. 2

FIG. 3

FIG. 4

FIG. 5

FIG. 6

FIG. 7

FIG. 8

RECOMMENDATION APPARATUS

FIG. 9

111 ATTRIBUTE INFORMATION

| PATIENT ID | GENDER | BODY FAT PERCENTAGE | BLOOD SUGAR LEVEL |
|---|---|---|---|
| 0001 | FEMALE | 28 | 118 |
| 0002 | MALE | 15 | 120 |
| . . . | . . . | . . . | . . . |

# FIG. 10

112 AREA INFORMATION

| AREA ID | GENDER | BODY FAT PERCENTAGE (BF) | BLOOD SUGAR LEVEL (BS) | CLASS |
|---|---|---|---|---|
| 0001 | FEMALE | $30 \leqq BF < 35$ | $120 \leqq BS < 130$ | NEGATIVE |
| 0002 | FEMALE | $15 \leqq BF < 30$ | $0 \leqq BS < 120$ | POSITIVE |
| ... | ... | ... | ... | ... |

# FIG. 11

{[GENDER = FEMALE], [30 ≤ BODY FAT PERCENTAGE < 35], [120 ≤ BLOOD SUGAR
LEVEL < 130], NEGATIVE}

FIG. 12

FIG. 13

MOVEMENT-TARGET DATA POINT
MOVEMENT PROCESS

START

S111
OBTAIN ONE CANDIDATE DESTINATION POINT THAT IS DISTANCE d AWAY IN POSITIVE DIRECTION

S112
CALCULATE DIFFERENTIALS FOR ITEM VALUES

S113
CALCULATE MAHALANOBIS DISTANCE USING EIGENVALUES AND EIGENVECTORS

S114
HAVE PREDETERMINED NUMBER OF CANDIDATE DESTINATION POINTS BEEN OBTAINED?

NO

YES

S115
MOVE MOVEMENT-TARGET DATA POINT TO CANDIDATE DESTINATION POINT WITH MINIMUM MAHALANOBIS DISTANCE

END

FIG. 14

FIG. 15

FIG. 16

BLOOD SUGAR LEVEL REDUCTION ACHIEVED

FIG. 17

FIG. 18

## INTERNATIONAL SEARCH REPORT

| International application No. |
|---|
| PCT/JP2020/025646 |

**A. CLASSIFICATION OF SUBJECT MATTER**
Int. Cl. G06N20/00(2019.01)i
FI: G06N20/00

According to International Patent Classification (IPC) or to both national classification and IPC

**B. FIELDS SEARCHED**

Minimum documentation searched (classification system followed by classification symbols)
Int. Cl. G06N20/00

Documentation searched other than minimum documentation to the extent that such documents are included in the fields searched
Published examined utility model applications of Japan     1922-1996
Published unexamined utility model applications of Japan   1971-2020
Registered utility model specifications of Japan           1996-2020
Published registered utility model applications of Japan   1994-2020

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2014-81878 A (HITACHI SYSTEMS, LTD.) 08 May 2014, entire text | 1-7 |
| A | JP 2003-288580 A (FUJITSU LTD.) 10 October 2003, entire text | 1-7 |

☐ Further documents are listed in the continuation of Box C.     ☒ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 15.09.2020 | 29.09.2020 |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**

Information on patent family members

| International application No. |
| --- |
| PCT/JP2020/025646 |

| Patent Documents referred to in the Report | Publication Date | Patent Family | Publication Date |
| --- | --- | --- | --- |
| JP 2014-81878 A | 08.05.2014 | (Family: none) | |
| JP 2003-288580 A | 10.10.2003 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003288580 A